(19) Europäisches Patentamt
European Patent Office
Office européen des brevets

(11) **EP 4 636 604 A1**

(12) **EUROPÄISCHE PATENTANMELDUNG**

(43) Veröffentlichungstag:
**22.10.2025  Patentblatt 2025/43**

(21) Anmeldenummer: **24170816.3**

(22) Anmeldetag: **17.04.2024**

(51) Internationale Patentklassifikation (IPC):
*G06F 13/12* (2006.01)   *G06F 13/16* (2006.01)
*G06F 13/28* (2006.01)   *G06F 13/40* (2006.01)
*A61B 6/00* (2024.01)   *A61B 8/08* (2006.01)
*G06T 11/00* (2006.01)   *A61B 6/03* (2006.01)
*G01T 1/29* (2006.01)

(52) Gemeinsame Patentklassifikation (CPC):
**G06F 13/28; A61B 6/5205; A61B 6/563;
A61B 6/566; G06F 13/128; G06F 13/1657;
G06F 13/1673; G06F 13/404; G06T 11/003;**
A61B 6/032; A61B 6/037

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(71) Anmelder: **Siemens Healthineers AG
91301 Forchheim (DE)**

(72) Erfinder:
• **Biele, Markus
91077 Hetzles (DE)**
• **Düppenbecker, Peter Michael
91074 Herzogenaurach (DE)**

• **Göderer, Edgar
91301 Forchheim (DE)**
• **Heinrich, Joerg
90768 Fürth (DE)**
• **Hosemann, Michael
91056 Erlangen (DE)**
• **Schwarz, Karl
91154 Roth (DE)**
• **Schweikert, Benjamin
91468 Gutenstetten (DE)**

(74) Vertreter: **Siemens Healthineers
Patent Attorneys
Postfach 22 16 34
80506 München (DE)**

(54) **BILDGEBUNGSGERÄT, BILDGEBUNGSSYSTEM UND VERFAHREN ZUM ÜBERTRAGEN VON MESSDATEN**

(57)   Die Erfindung betrifft ein Bildgebungssystem umfassend ein Bildgebungsgerät mit einer Erfassungsvorrichtung (1) zum Erfassen und Weiterleiten von Messdaten, wobei die Erfassungsvorrichtung (1) zumindest ein Erfassungsmodul, insbesondere eine Mehrzahl von Erfassungsmodulen, zum Erfassen von Teildaten, die jeweils ein Teil der gesamten Messdaten sind, umfasst, wobei das Bildgebungsgerät dazu konfiguriert ist, mit Hilfe des zumindest einen Erfassungsmoduls Gruppen von Teildaten zu erzeugen, wobei jede der Gruppen Teildaten von zumindest einem des zumindest einen Erfassungsmoduls umfasst; wobei das Bildgebungssystem zumindest einen Adressierungsalgorithmus umfasst, der dazu konfiguriert ist, basierend auf Informationen über die Struktur einer Datenverarbeitungshardware mit einer Mehrzahl von Zielspeicherbereichen, optional verteilt auf mehrere Zielspeicher, die Gruppen jeweils mit einer Zieladresse (7) betreffend jeweils einen der Zielspeicherbereiche in der Datenverarbeitungshardware zu verknüpfen; wobei das Bildgebungsgerät zumindest eine Ausgabeschnittstelle zum Weiterleiten der mit jeweils zumindest einer Zieladresse (7) verknüpften Gruppen von Teildaten in Richtung zumindest des der Zieladresse (7) entsprechenden Zielspeicherbereichs umfasst.

FIG 1

**Beschreibung**

Unabhängig vom grammatikalischen Geschlecht eines bestimmten Begriffes sind Personen mit männlicher, weiblicher oder anderer Geschlechteridentität mit umfasst.

Die Erfindung betrifft ein Bildgebungssystem und ein Verfahren zum Übertragen von Messdaten.

Beim Betrieb von Bildgebungsgeräten, insbesondere medizinischen Bildgebungsgeräten, werden oftmals große Mengen von Messdaten erfasst, die typischerweise im Folgenden weiterverarbeitet werden sollen, insbesondere im Rahmen einer Bildrekonstruktion. Ein technisches Problem dabei ist die Datenübertragung der Messdaten. Beispielsweise müssen bei einem Computertomographiesystem Messdaten von einer Datenquelle in einem Röntgendetektor zu einem Bildrekonstruktionssystem übertragen werden, damit sie dort verarbeitet und rekonstruiert werden können. Ein Problem ist auch die effiziente Koordination der Weiterverarbeitung der oftmals großen Mengen an Messdatendaten.

Um dieses Problem zu lösen, gibt es bereits einige Ansätze im Rahmen von Bildgebungssystemen. Beispielsweise können die Daten über einen Ethernet-Link an einen Empfänger versendet werden und dann zunächst auf einem Speicher eines Bildrekonstruktionssystems gespeichert werden, bevor sie auf dem Bildrekonstruktionssystem weiterverarbeitet werden. Beispielsweise können bei einem Computertomographiesystem mit einer rotierenden Gantry die Daten über den Ethernet-Link auf einen stationären Teil des Bildgebungssystems geleitet werden, an dem sich das Bildrekonstruktionssystem befinden kann oder an den das Bildrekonstruktionssystem angeschlossen sein kann. Typischerweise umfasst diese Datenübertragung mehrere weitere Zwischenschritte, die beispielsweise jeweils ein Codieren, Sortieren und/oder Zuordnen der Daten auf ihren jeweiligen Stationen, bzw. bei den jeweiligen Komponenten des Bildgebungssystems, umfassen können.

Es ist daher eine Aufgabe der vorliegenden Erfindung, eine effizientere Möglichkeit zur Datenübertragung von einer Datenquelle zu einer Hardware zur weiteren Datenverarbeitung und gegebenenfalls auch zur Weiterverarbeitung, insbesondere Rekonstruktion, der Daten bereitzustellen.

Diese Aufgabe wird gelöst durch ein Bildgebungssystem gemäß Anspruch 1 und ein Verfahren gemäß Anspruch 15. Weitere Merkmale und Vorteile ergeben sich aus den abhängigen Ansprüchen, der Beschreibung und den beigefügten Figuren.

Gemäß einem ersten Aspekt der Erfindung ist ein Bildgebungssystem umfassend ein Bildgebungsgerät, insbesondere Computertomographiegerät, Positronen-Emissions-Tomographie-Gerät, Fluoroskopie-Gerät und/oder Ultraschall-Gerät, wobei das Bildgebungsgerät eine Erfassungsvorrichtung zum Erfassen und Weiterleiten von Messdaten umfasst, vorgesehen. Die Erfassungsvorrichtung umfasst zumindest ein Erfassungsmodul, insbesondere eine Mehrzahl von Erfassungsmodulen, zum Erfassen von Teildaten, die jeweils ein Teil der gesamten Messdaten sind, wobei das Bildgebungsgerät dazu konfiguriert ist, mit Hilfe des zumindest einen Erfassungsmoduls Gruppen von Teildaten zu erzeugen, wobei jede der Gruppen Teildaten von zumindest einem des zumindest einen Erfassungsmoduls umfasst. Das Bildgebungssystem umfasst zumindest einen Adressierungsalgorithmus, der dazu konfiguriert ist, basierend auf Informationen über die Struktur einer Datenverarbeitungshardware mit einer Mehrzahl von Zielspeicherbereichen, optional verteilt auf mehrere Zielspeichern, die Gruppen jeweils mit einer Zieladresse betreffend jeweils einen der Zielspeicherbereiche in der Datenverarbeitungshardware zu verknüpfen. Das Bildgebungsgerät umfasst zumindest eine Ausgabeschnittstelle zum Weiterleiten der mit jeweils zumindest einer Zieladresse verknüpften Gruppen von Teildaten in Richtung zumindest des der Zieladresse entsprechenden Zielspeicherbereichs. Vorteilhafterweise kann durch mit dem erfindungsgemäßen Bildgebungssystem, insbesondere mit Hilfe des Adressierungsalgorithmus, eine deutliche Vereinfachung der Datenübertragung erzielbar sein. Beispielsweise kann es vorgesehen sein, dass einzelne Komponenten zwischen der Ausgabeschnittstelle und dem jeweiligen Zielspeicherbereich der Datenverarbeitungshardware die (Teil-)Daten lediglich durchleiten müssen, wobei durch die Zieladresse festgelegt ist, wohin die Daten geleitet werden sollen. Insbesondere können für das Durchleiten der Daten auch Standardkomponenten und/oder Standardprotokolle vorgesehen sein. Mit Standardkomponenten bzw. Standardprotokollen kann ein Aufbau eines Bildgebungssystems kostengünstiger möglich sein und auch ein Austausch von Komponenten kann vereinfacht sein. Dadurch, dass den jeweiligen Teildaten bereits frühzeitig eine Zieladresse zugewiesen ist, kann eine reduzierte Anzahl an Transfers zwischen verschiedenen Speichern erfolgen. Im Stand der Technik werden die Messdaten dagegen typischerweise erst in der Datenverarbeitungshardware dem jeweiligen Zielspeicherbereich zugewiesen, was oftmals mit mehreren Transfers zwischen verschiedenen Komponenten der Datenverarbeitungshardware, z.B. zwischen Grafikprozessoren, der Prozessoreinheit (CPU) und oder dem RAM verbunden ist. Insbesondere bei steigenden Datenmengen (z.B. aufgrund von photonenzählenden Röntgendetektoren in Computertomographiesystem, höherer Auflösung und größeren Detektoren) kann mit dem erfindungsgemäßen Adressierungsalgorithmus eine Vereinfachung der Datenübertragungskette erzielt werden und somit die Zahl von Sende-, Empfangs- und Kopieroperationen reduziert werden. Somit kann eine effizientere Verarbeitungsarchitektur und eine verschlanke Übertragungskette, insbesondere mit erheblicher Komplexitäts-, Kosten- und Energiereduktion, erzielt werden.

Das Bildgebungsgerät kann insbesondere ein medizinisches Bildgebungsgerät sein. Beispielsweise kann das Bildgebungsgerät ein Computertomographiegerät, ein Positronen-Emissions-Tomographie-Gerät, ein Fluoroskopie-Gerät und/oder ein Ultraschall-Gerät sein. Aspekte der Erfindung, insbesondere betreffend eine Adressierung von Teildaten der Messdaten und die Übertragung der Teildaten kann im Rahmen eines Computertomographiegeräts besonders vorteilhaft sein. Die erfindungsgemäßen Aspekte können jedoch auch für andere Bildgebungsgeräte und Bildgebungssysteme, insbesondere solche mit einer hohen Datenübertragungsrate und unterteilbaren Erfassungsmodulen, wie ein Positronen-Emissions-Tomographie-Gerät, ein Fluoroskopie-Gerät und/oder ein Ultraschall-Gerät, vorteilhaft sein. Die Erfassungsvorrichtung kann beispielsweise ein Röntgendetektor sein und/oder einen Röntgendetektor umfassen. Die Erfassungsmodule können beispielsweise Detektormodule des Röntgendetektor sein. Insbesondere kann das Computertomographiegerät eine rotierbare Gantry umfassen, wobei der Röntgendetektor dazu ausgestaltet ist, während einer Messung um einen Untersuchungsbereich zu rotieren. In einem Röntgendetektor eines Computertomographiegeräts sind die Erfassungsmodule bzw. Detektormodule typischerweise räumlich verteilt. Im Stand der Technik ist es üblich, die Daten der verschiedenen Erfassungsmodule zusammenzufassen und gemeinsam weiterzuleiten. Demgegenüber ist erfindungsgemäß bereits frühzeitig eine gezielte Adressierung von Teildaten vorgesehen.

Das Bildgebungsgerät ist dazu konfiguriert, mit Hilfe des zumindest einen Erfassungsmoduls Gruppen von Teildaten zu erzeugen. Dabei umfasst jede der Gruppen Teildaten von zumindest einem Erfassungsmodul. Die Gruppen können gemäß vorbestimmten Kriterien eingeteilt sein. Es kann vorgesehen sein das im Wesentlichen jede der Gruppen, insbesondere genau jeweils eine der Gruppen, Teildaten von genau einem Erfassungsmodul umfassen. Es kann vorgesehen sein, dass eine, einzelne, mehrere oder alle der Gruppen Teildaten von jeweils mehreren Erfassungsmodulen umfassen. Es kann vorgesehen sein, dass von einem Erfassungsmodul mehrere Teildaten stammen. Es kann optional eine Adressierung innerhalb bestimmter Bereiche vorgesehen sein. Beispielsweise können mehreren Datenausgängen, jeweils räumliche Teilbereiche zugeordnet sein. Die Gruppen können nach messtechnischen Kriterien eingeteilt sein. Beispielsweise können die Gruppen basierend auf einem Messort, insbesondere in einem Untersuchungsobjekt und oder in einem Untersuchungsbereich, von dem die erfassten Daten stammen, eingeteilt sein. Die Gruppen können so eingeteilt sein, dass die Teildaten, die von der Datenverarbeitungshardware, insbesondere in einem ersten Verarbeitungsschritt, zusammen verarbeitet werden sollen, jeweils einer gemeinsamen Gruppe von Teildaten angehören. Vorteilhafterweise kann durch das Erzeugen der Gruppen eine effizientere Verarbeitung der Messdaten gefördert werden.

Das Bildgebungssystem umfasst zumindest einen Adressierungsalgorithmus. Optional kann es vorgesehen sein, dass das Bildgebungsgerät den Adressierungsalgorithmus umfasst. Es kann jedoch auch vorgesehen sein, dass der Adressierungsalgorithmus außerhalb des Bildgebungsgeräts, beispielsweise auf der Datenverarbeitungshardware, vorgesehen ist. Der zumindest eine Adressierungsalgorithmus kann in die Erfassungsvorrichtung integriert sein. Insbesondere kann der Adressierungsalgorithmus in einem Frontend der Erfassungsvorrichtung und/oder des Bildgebungsgeräts integriert sein. Es kann vorgesehen sein, dass je ein Adressierungsalgorithmus jeweils einem Erfassungsmodul oder jeweils einer Gruppe von Erfassungsmodulen zugeteilt ist. Der Adressierungsalgorithmus ist dazu konfiguriert, basierend auf Informationen über die Struktur einer Datenverarbeitungshardware mit einer Mehrzahl von Zielspeicherbereichen, die Gruppen jeweils mit einer Zieladresse betreffend jeweils einen der Zielspeicherbereiche in der Datenverarbeitungshardware zu verknüpfen. Die Zielspeicherbereiche sind insbesondere Teil zumindest eines Zielspeichers. Die Datenverarbeitungshardware kann insbesondere dazu ausgestaltet sein die Messdaten zu verarbeiten. Beispielsweise kann die Datenverarbeitungshardware dazu ausgestaltet sein, eine Bildrekonstruktion durchzuführen. Die Datenverarbeitungshardware kann insbesondere einen Grafikprozessor (GPU) umfassen. Die Mehrzahl von Zielspeicherbereichen kann beispielsweise Teil des Grafikspeichers sein. Alternativ oder zusätzlich kann die Mehrzahl von Zielspeicherbereichen beispielsweise Teil eines Arbeitsspeichers sein. Es können mehrere Zielspeicher, insbesondere jeweils mit einem oder mehreren Zielspeicherbereichen vorgesehen sein. Beispielsweise können mehrere Zielspeicher verschiedener Grafikprozessoren vorgesehen sein. Der Adressierungsalgorithmus kann Informationen über die Struktur der Datenverarbeitungshardware umfassen und/oder auf Informationen über die Struktur der Datenverarbeitungshardware Zugriff haben. Beispielsweise können die Informationen eine Auflistung von verfügbaren Zielspeichern und/oder Zielspeicherbereichen umfassen. Die Informationen können eine Größe des oder der jeweiligen Zielspeicher(s) bzw. der Zielspeicherbereiche umfassen. Die Informationen können eine Verarbeitungsdauer an dem jeweiligen Zielspeicherbereich umfassen. Die Informationen können eine relative Positionierung der Zielspeicherbereiche, insbesondere verschiedener Zielspeicher, zueinander umfassen. Beispielsweise können die Informationen eine Zuordnung verschiedener Zielspeicher zu verschiedenen Komponenten der Datenverarbeitungshardware, beispielsweise zu verschiedenen Grafikprozessoren, umfassen.

Das Bildgebungsgerät umfasst zumindest eine Ausgabeschnittstelle zum Weiterleiten der mit jeweils zumindest einer Zieladresse verknüpften Gruppen von Teildaten in Richtung zumindest des der Zieladresse entsprechenden Zielspeicherbereichs. Der Begriff Ausgabeschnittstelle ist im Rahmen dieser Erfindung breit zu verstehen. Generell bezeichnet eine Ausgabeschnittstelle eine Schnittstelle die dazu konfiguriert ist, die Teildaten, insbesondere an zumindest eine weitere Komponente, weiterzuleiten. Die weitere Komponente kann direkt die Datenverarbeitungshardware sein. Es kann aber auch vorgesehen sein, dass zwischen der Datenverarbeitungshardware und der Ausgabe Schnittstelle zumindest eine weitere Komponente ist. Optional kann es vorgesehen sein, dass die Datenverarbeitungshardware selbst Teil des Bildgebungsgeräts und/oder des Bildgebungssystems ist. Alternativ kann die Datenverarbeitungshardware auch extern von dem Bildgebungsgerät vorgesehen sein. Die Ausgabeschnittstelle kann beispielsweise dazu ausgestaltet sein, die Messdaten über eine kabelbasierende Verbindung, insbesondere Ethernet-Verbindung, weiterzuleiten. Bei einer rotierenden Erfassungsvorrichtung kann eine kabelbasierende Verbindung beispielsweise einen Schleifkontakt umfassen. Alternativ oder zusätzlich kann die Ausgabeschnittstelle dazu ausgestaltet sein, die Messdaten über eine drahtlose Verbindung weiterzuleiten.

Gemäß einer Ausführungsform umfasst das Bildgebungsgerät eine Mehrzahl von integrierten Schaltkreisen, insbesondere anwendungsspezifischen integrierten Schaltungen, die jeweils zumindest einem Erfassungsmodul zugeordnet sind und dazu konfiguriert sind, Teildaten von dem zumindest einen zugeordneten Erfassungsmodul zu verarbeiten, wobei die Mehrzahl von integrierten Schaltkreisen jeweils einen des zumindest einen Adressierungsalgorithmus umfassen, oder wobei der zumindest eine Adressierungsalgorithmus Teil zumindest einer den integrierten Schaltkreisen unmittelbar nachgelagerten Verarbeitungsstufe in dem Bildgebungsgerät ist. Der Begriff "anwendungsspezifische integrierte Schaltung" (englisch application-specific integrated circuit, ASIC) ist im Rahmen dieser Erfindung breit zu verstehen und bezeichnet allgemein eine elektronische Schaltung, die als integrierter Schaltkreis realisiert ist. Die Mehrzahl von integrierten Schaltkreisen können dazu ausgestaltet sein, die Messdaten bzw. jeweils die Teildaten basierend auf einem Signaleingang der Erfassungsmodule zu erzeugen. Insbesondere können die integrierten Schaltkreise der Mehrzahl von integrierten Schaltkreisen jeweils im Wesentlichen unmittelbar an einen Signaleingang des jeweiligen Erfassungsmoduls angebunden sein. Typischerweise ist eine Vielzahl von anwendungsspezifischen integrierten Schaltungen (ASICs) in einem Computertomographiesystem vorgesehen. Es können beispielsweise in der Größenordnung von 1000, zum Beispiel 500-5000, anwendungsspezifischen integrierten Schaltungen (ASICs) vorgesehen sein. Indem bereits in den integrierten Schaltkreisen oder zumindest in der den integrierten Schaltkreisen unmittelbar nachgelagerten Verarbeitungsstufe jeweils ein Adressierungsalgorithmus vorgesehen ist, kann den Teildaten frühzeitig die jeweilige Zieladresse zugeordnet werden. Vorteilhafterweise müssen somit weitere nachgelagerte Komponenten lediglich die Teildaten gemäß der Zieladresse weiterleiten und ein neues Adressieren der Messdaten in späteren Verarbeitungsstufen kann umgangen werden. Somit können mehrere Zwischenschritte, in denen eine (erneute) Adressierung der Messdaten nötig wäre umgangen werden. Insbesondere kann eine Weiterverarbeitung mit einer möglichst geringen Anzahl an Transfers zwischen verschiedenen Speichern ermöglicht werden. Ist der Adressierungsalgorithmus jeweils in den den Erfassungsmodulen zugeordneten integrierten Schaltkreisen, insbesondere anwendungsspezifischen integrierten Schaltungen, vorgesehen, kann dies besonders vorteilhaft sein, weil somit eine Adressierung mit der Zieladresse bereits noch früher geschehen kann. Insbesondere kann somit beispielsweise ein weiterer Zwischenschritt umgangen werden und/oder eine benötigte Bandbreite von dem integrierten Schaltkreis zu der nächsten Verarbeitungsstufe kann reduziert werden. Eine unmittelbar nachgelagerten Verarbeitungsstufe kann beispielsweise ein (ggf. weiterer) dedizierter anwendungsspezifischer integrierter Schaltkreis (dedizierter ASIC), ein SoC (System-on-Chip), ein SiP (System-in-Package), ein FPGA (Field Programmable Gate Array), ein DSP (digitaler Signalprozessor), eine CPU (Central Processing Unit) oder etwas Entsprechendes sein. Den Adressierungsalgorithmus in einer nachgelagerten Verarbeitungsstufe vorzusehen kann den Vorteil haben, dass es typischerweise einfacher ist einen Code des Adressierungsalgorithmus noch anzupassen und zu testen. Beispielsweise kann ein Adressierungsalgorithmus in der FPGA noch im Einsatz getestet und entwickelt werden, bis er wie gewünscht funktioniert.

Gemäß einer Ausführungsform umfasst das Bildgebungsgerät zumindest einen Gruppierungsalgorithmus, der dazu konfiguriert ist, die Teildaten verschiedener Erfassungsmodule und/oder verschiedener Erfassungszeitpunkte zu den Gruppen von Teildaten zusammenzufassen, insbesondere bevor die Gruppen von dem Adressierungsalgorithmus mit einer Zieladresse verknüpft werden. Das Zusammenfassen kann beispielsweise auf einer $2\times2$-Pixel-Zusammenfassung, auf einem Spectral-Mixing, etc., basieren. Der zumindest eine Gruppierungsalgorithmus kann insbesondere jeweils in der Mehrzahl von integrierten Schaltkreisen, insbesondere anwendungsspezifischen integrierten Schaltungen, oder der zumindest einen dem integrierten Schaltkreis unmittelbar nachgelagerten Verarbeitungsstufe vorgesehen sein. Der Gruppierungsalgorithmus kann beispielsweise dazu konfiguriert sein, Teildaten so zusammenzufassen, dass Daten gemäß einer logischen und/oder räumlichen Zusammengehörigkeit und/oder basierend auf einem zeitlichen Zusammenhang der Daten, insbesondere der Teildaten, zusammengefasst werden. Eine logische Zusammengehörigkeit der Daten kann beispielsweise basierend darauf erfolgen, welche Daten in der Datenverarbeitungshardware, insbesondere in einem ersten Verarbeitungsschritt, gemeinsam verarbeitet werden sollen. Eine räumliche Zusammengehörigkeit kann beispielsweise darauf basieren, dass Daten von einem gemeinsamen Messort, insbesondere in einem Untersuchungsobjekt und oder in einem Untersuchungsbereich, stammen. Das Berücksichtigen eines zeitlichen Zusammenhangs der Daten kann insbesondere hilfreich sein, wenn ein zeitlicher Zusammenhang von Daten verschiedener Detektoren relevant ist, wie z.B. in der Positronen-Emissions-Tomographie. Der Gruppierungsalgorithmus kann dazu konfiguriert sein, Messdaten eines oder mehrerer Aufnahme-Frames zusammenzufassen. Beispielsweise kann der Gruppierungsalgorithmus dazu konfiguriert sein, 4 bis 128, bevorzugt 8 bis 64 Aufnahme-Frames, zusammenzufassen. Vorteilhafterweise kann mit dem Gruppierungsalgorithmus eine effiziente Verarbeitung der Messdaten gefördert werden.

Gemäß einer Ausführungsform umfasst das Bildgebungsgerät zumindest einen Komprimierungsalgorithmus, der dazu konfiguriert ist, Gruppen von Teildaten zu komprimieren, bevor diese an die Ausgabeschnittstelle übergeben werden, insbesondere bevor die Gruppen von dem Adressierungsalgorithmus mit einer Zieladresse verknüpft werden. Der zumindest eine Komprimierungsalgorithmus kann insbesondere jeweils in der Mehrzahl von integrierten Schaltkreisen, insbesondere anwendungsspezifischen integrierten Schaltungen, oder der zumindest einen dem integrierten Schaltkreis unmittelbar nachgelagerten Verarbeitungsstufe vorgesehen sein. Der Komprimierungsalgorithmus kann dazu ausgestaltet sein, die von dem Gruppierungsalgorithmus zusammengefassten Teildaten zu komprimieren. Beispielsweise kann der Komprimierungsalgorithmus auf einer differenziellen und/oder Lauflängenkodierung basieren. Optional kann es vorgesehen sein, dass der Komprimierungsalgorithmus eine trainierte künstliche Intelligenz umfasst. Beispielsweise kann der Komprimierungsalgorithmus analog wie in DE 10 2010 063 435 A1 beschrieben ausgeführt sein.

Gemäß einer Ausführungsform ist der Adressierungsalgorithmus ferner dazu konfiguriert, die jeweilige Zieladresse auch basierend auf zumindest einer zeitlichen Information betreffend die Teildaten festzulegen. Die zumindest eine zeitliche Information kann insbesondere einen zeitlichen Zusammenhang verschiedener Teildaten betreffen. Das Berücksichtigen einer zeitlichen Information kann insbesondere hilfreich sein, wenn ein zeitlicher Zusammenhang von Daten verschiedener Detektoren relevant ist, wie z.B. in der Positronen-Emissions-Tomographie.

Gemäß einer Ausführungsform ist die Erfassungsvorrichtung dazu ausgestaltet, während der Erfassung von Messdaten bewegt zu werden, insbesondere rotiert zu werden, wobei der zumindest eine Adressierungsalgorithmus ferner dazu konfiguriert ist, die jeweilige Zieladresse auch basierend auf der aktuellen Position der Erfassungsmodule, von denen die jeweiligen Teildaten stammen, festzulegen. Der Adressierungsalgorithmus kann Informationen über eine relative Position des jeweiligen Erfassungsmoduls in der Erfassungsvorrichtung umfassen und/oder dazu ausgestaltet sein, die relative Position des jeweiligen Erfassungsmoduls in der Erfassungsvorrichtung zu empfangen. Das Bildgebungsgerät kann dazu ausgestaltet sein, eine aktuelle Position der Erfassungsvorrichtung und/oder der Erfassungsmodule zu bestimmen und an den zumindest ein Adressierungsalgorithmus weiterzugeben. Beispielsweise kann der Adressierungsalgorithmus dazu konfiguriert sein, anhand der aktuellen Position der Erfassungsvorrichtung und anhand der Information über die relative Position des jeweiligen Erfassungsmoduls in der Erfassungsvorrichtung die aktuelle Position des Erfassungsmoduls zu ermitteln. Durch eine Positionsabhängigkeit der Zieladresse kann erreicht werden, dass die Teildaten jeweils an einen für die Weiterverarbeitung optimiertem Zielspeicherbereich bzw. Zielspeicher gelangen. Der jeweilige Zielspeicherbereich bzw. Zielspeicher kann beispielsweise konkret dafür ausgestaltet sein, Teildaten von einer bestimmten Messposition zu verarbeiten. Beispielsweise kann es so möglich sein, dass die Datenverarbeitungshardware basierend auf einem Scanprotokoll automatisch eine Verarbeitung der Daten vornimmt, ohne dass eine Adressierung der Daten in der Datenverarbeitung Hardware selbst vorgenommen werden muss. Die Datenverarbeitungshardware kann somit aufgrund der Zieladressen, die den Teildaten bereits zugeordnet sind, im Wesentlichen direkt mit der Datenverarbeitung beginnen, wobei eine Zuordnung der Teildaten bereits durch den Adressierungsalgorithmus vorgegeben ist. Beispielsweise kann die Erfassungsvorrichtung ein rotierbarer Röntgendetektor, insbesondere eines Computertomographiesystems, sein. Bei einem Computertomographiesystem mit einem rotierbaren Röntgendetektor müssen typischerweise Daten aus verschiedenen räumlichen Regionen zusammengefasst werden. Durch die Rotation kann eine ähnliche physikalische Position von unterschiedlichen Erfassungsmodulen bzw. auch unterschiedlichen daran angeschlossenen integrierten Schaltkreisen stammen. Beispielsweise kann es zweckmäßig sein, Daten von einer bestimmten Rotationsposition, wie beispielsweise der 9-Uhr-Position, immer an einen gleichen Zielspeicherbereichen oder an ein gleiches Set von Zielspeicherbereichen zu adressieren. Entsprechend kann der jeweilige Empfänger der Datenverarbeitungshardware davon ausgehen, dass die Teildaten, die er erhält, jeweils von einer bestimmten Position stammen, sodass keine weitere Zuordnung nötig ist.

Gemäß einer Ausführungsform ist der Adressierungsalgorithmus ferner dazu konfiguriert, die jeweilige Zieladresse auch basierend auf einer logischen und/oder physischen Position der Zielspeicherbereiche, insbesondere mehrerer Zielspeicher, in der Datenverarbeitungshardware festzulegen. Insbesondere kann es vorgesehen sein, dass die Datenverarbeitungshardware mehrere Hardwarekomponenten umfasst, und der Adressierungsalgorithmus in der jeweiligen Zieladresse eine bestimmte der mehreren Hardwarekomponenten adressiert. Mehrere Hardwarekomponenten können beispielsweise mehrere Grafikprozessoren sein oder umfassen. Entsprechend kann eine physische Position beispielsweise eine Position des Zielspeicherbereichs bzw. Zielspeichers in jeweils einer von mehreren Hardwarekomponenten sein. Es kann beispielsweise vorgesehen sein, dass verschiedene Teildaten, die anfangs oder in einem späteren Schritt gemeinsam verarbeitet werden sollen, zu einem Zielspeicherbereich bzw. Zielspeicher jeweils der gleichen Hardwarekomponente, beispielsweise des gleichen Grafikprozessors, adressiert werden. Eine logische Position kann beispielsweise eine Zieladresse in der Datenverarbeitungshardware sein, die dazu konfiguriert ist, eine bestimmte Art von Daten, beispielsweise Daten von einer bestimmten Position oder Daten, die gemäß einem bestimmten Parameter aufgenommen wurden, weiterzuverarbeiten. Ein bestimmter Parameter kann beispielsweise eine Energieschwelle, eine verwendete Spannung und/oder eine bestimmte Frequenz etc. sein. Eine logische Position kann optional auch eine Position sein, die gemäß in dem Adressierungsalgorithmus hinterlegten Informationen und basierend auf den zuvor versendeten Teildaten und deren Zieladressen erwartungsgemäß aktuell frei für die Aufnahme von weiteren Teildaten ist.

Gemäß einer Ausführungsform ist der zumindest eine Adressierungsalgorithmus dazu konfiguriert, die Zieladressen jeweils auch basierend auf Informationen über eine Verarbeitungsdauer der Datenverarbeitungshardware an dem jeweiligen Zielspeicherbereich festzulegen, sodass das erneute Senden von Teildaten in einen Zielspeicherbereich mit dem Freiwerden des Zielspeicherbereichs abgestimmt ist. Insbesondere kann mit dieser Ausführungsform einerseits erreicht werden, dass sich Teildaten von aufeinanderfolgenden Übertragungen nicht überschreiben und dass andererseits eine möglichst effiziente Nutzung der Datenverarbeitungshardware ermöglicht wird, indem Rechenleistung nicht durch temporär zu lange leer bleibenden Zielspeicherbereiche ungenutzt bleibt. Der Adressierungsalgorithmus kann somit dazu konfiguriert sein, den Zeitpunkt und gegebenenfalls die Art der zuvor gesendeten Teildaten mit der Verarbeitungsdauer an der jeweiligen Zieladresse gemeinsam zu berücksichtigen, um ein Nachfüllen der Zielspeicherbereiche mit neuen Teildaten gezielt zeitmäßig zu takten. Ermöglicht beispielsweise der Speicher eines Grafikprozessors der Datenverarbeitungshardware die Speicherung der Daten zweier kompletter Rotationen einer rotierbaren Erfassungsvorrichtung, so kann es vorgesehen sein, bei einer Rotationszeit von 0,2 Sekunden den gleiche Speicherbereich erst nach 400 Millisekunden erneut zu überschreiben. Entsprechend sollte in diesem Zeitfenster die Weiterverarbeitung der Teildaten in dem jeweiligen Block des Zielspeichers sicher abgeschlossen sein.

Gemäß einer Ausführungsform sind das Bildgebungssystem, insbesondere das Bildgebungsgerät, und der Adressierungsalgorithmus so konfiguriert, dass der Adressierungsalgorithmus durch eine Eingabe parametrisierbar ist, wobei die Parametrisierbarkeit insbesondere in Abhängigkeit von vorgesehenen Messparametern eines Messprotokolls vorgesehen sein kann. Der Adressierungsalgorithmus kann beispielsweise verschiedene Konfigurationsregister aufweisen. Beispielsweise kann der Adressierungsalgorithmus in verschiedene Operationsmodi, die insbesondere jeweils einer verschiedenen Parametrisierung entsprechen, einstellbar sein. Die Parametrisierbarkeit kann das Einstellen von verschiedenen Übertragungsmodi der Messdaten bzw. Teildaten umfassen. Die Messparameter können beispielsweise eine Rotationszeit, eine Framerate, eine Z-Abdeckung, eine Schwellenzahl und/oder Ortsauflösung umfassen. Die Parametrisierbarkeit kann auf zumindest eine Eigenschaft der Datenverarbeitungshardware abgestimmt sein. Beispielsweise kann die Parametrisierbarkeit auf eine Systemkonfiguration der Datenverarbeitungshardware abgestimmt sein. Eine Systemkonfiguration kann beispielsweise eine Anzahl, Art, und/oder Ausbaustufe von Grafikprozessoren der Datenverarbeitungshardware umfassen. Beispielsweise kann der Adressierungsalgorithmus verschiedene Operationsmodi abgestimmt auf die Eigenschaft, insbesondere Systemkonfiguration, der Datenverarbeitungshardware umfassen. Durch die Parametrisierbarkeit kann das Bildgebungssystem und der Adressierungsalgorithmus flexibler, insbesondere in verschiedenen Kontexten, mit verschiedener Datenverarbeitungshardware und/oder in verschiedenen Messmodi, eingesetzt werden. Beispielsweise kann im Rahmen eines Computertomographiesystems, ein Übergang zwischen Dual-Source und Single-Source Systemen im Wesentlichen nur eine Umparametrisierung des Algorithmus erfordern. Eine Erhöhung einer Schwellenanzahl der Detektormodule des Computertomographiesystems kann im Wesentlichen durch die Anpassungen der Mehrzahl von integrierten Schaltkreisen, insbesondere anwendungsspezifischen integrierten Schaltungen, der Parametrisierung des Adressierungsalgorithmus und der Verarbeitung in der Datenverarbeitungshardware, insbesondere der Grafikprozessor-Internen Verarbeitung, erfolgen. Insbesondere bei ausreichend Bandbreite und Verarbeitungskapazität kann dies mit geringen Auswirkungen auf andere Komponenten erzielbar sein. Mit anderen Worten müssen somit ggf. nur die Endkomponenten angepasst werden, während alle Komponenten dazwischen unbeeinflusst bleiben können. In aktuellen Systemen gemäß dem Stand der Technik würde eine solche Umstellung dagegen die Anpassung oder den Austausch quasi jeder Komponente auf dem Datenübertragungsweg erfordern. Vorteilhafterweise kann durch die Parametrisierbarkeit beispielsweise ein Seitenkanal bei der Datenübertragung der Teildaten zwischen Sender und Empfänger weitgehend entfallen, weil eine Synchronisierung über den Seitenkanal gegebenenfalls nicht mehr nötig ist. Dies kann dadurch ermöglicht werden, dass durch die Parametrisierbarkeit die Daten bereits passend abgestimmt sein können, auch für verschiedene Arten von Datenverarbeitungshardware oder verschiedene Messmodi. Vorteilhafterweise kann somit die Verarbeitungsarchitektur noch effizienter und mit reduzierter Komplexität ermöglicht werden. Auch können Kosten und ein Energieverbrauch reduziert werden.

Gemäß einer Ausführungsform ist bzw. sind das Bildgebungsgerät und/oder die zumindest eine Ausgabeschnittstelle dazu konfiguriert, die Gruppen von Teildaten jeweils in Richtung zumindest eines zusätzlichen Datenspeichers weiterzuleiten. Vorteilhafterweise können die Messdaten somit, beispielsweise für eine spätere Weiterverarbeitung oder Überprüfung, auch als Rohdaten gespeichert werden.

Gemäß einer Ausführungsform umfasst das Bildgebungssystem eine Datenverarbeitungshardware mit einer Mehrzahl von Zielspeicherbereichen, optional verteilt auf mehrere Zielspeicher, wobei die Datenverarbeitungshardware insbesondere zumindest einen Grafikprozessor umfasst, wobei die Datenverarbeitungshardware mit der zumindest einen Ausgabeschnittstelle datentechnisch verbunden und/oder verbindbar ist und dazu konfiguriert ist, die Gruppen von Teildaten zu verarbeiten, insbesondere im Rahmen einer Bildrekonstruktion. Die Mehrzahl von Zielspeicherbereichen kann beispielsweise Teil des Grafikspeichers sein. Die Datenverarbeitungshardware kann optional mehrere Grafikprozessoren umfassen. Die Datenverarbeitungshardware kann insbesondere dazu ausgestaltet sein, basierend auf den Messdaten eine Bildrekonstruktion durchzuführen. Der zumindest eine Grafikprozessor kann dazu ausgestaltet sein, eine Datenrekonstruktion intern durchzuführen und erst ein vollständig rekonstruiertes Bild, beispielsweise ein rekonstruiertes Schnittbild, auszugeben. Die Datenverarbeitungshardware ist dazu ausgestaltet, eingehende Teildaten weiterzuverarbeiten. Insbesondere kann die Datenverarbeitungshardware so ausgestaltet sein, dass die vollständige Weiterverarbeitung innerhalb zumindest eines Grafikprozessors durchgeführt wird. Die Datenverarbeitungshardware kann dazu ausgestaltet sein, eingehende Teildaten zu dekomprimieren und anhand einer Vorverarbeitungsstufe vorzubereiten. Das Dekomprimieren und die Vorverarbeitungsstufe können vorzugsweise bereits Teil der Weiterverarbeitung innerhalb des zumindest einen Grafikprozessors sein. Die Vorverarbeitungsstufe kann beispielsweise ein Anwenden von Korrekturen auf die Teildaten umfassen. Die Vorverarbeitungsstufe kann auch als Prep-Kette bezeichnet werden. Nach der Vorverarbeitungsstufe ist insbesondere zumindest eine weitere Verarbeitungsstufe vorgesehen. Insbesondere kann die zumindest eine weitere Verarbeitungsstufen zum Durchführen einer Bildrekonstruktion ausgestaltet sein. Die zumindest eine weitere Verarbeitungsstufe kann zumindest eine Rebinning-Stufe umfassen, insbesondere zum Überführen der in der Vorverarbeitungsstufe vorbereiteten Daten in zumindest einen Satz von Projektionen.

Gemäß einer Ausführungsform ist die Datenverarbeitungshardware dazu konfiguriert, die Gruppen von Teildaten anhand der verknüpften Zieladressen direkt an den zugehörigen Zielspeicherbereich zu leiten und die Teildaten an dem Ort des Zielspeicherbereichs zu bearbeiten, wobei die Datenverarbeitungshardware optional dazu konfiguriert ist, die Teildaten nach der Bearbeitung in dem Zielspeicherbereich an einen weiteren Speicherort der Datenverarbeitungshardware weiterzugeben und dort weiterzubearbeiten. Zusätzlich zu dem einen weiteren Speicherort können eine oder mehrere noch weitere Speicherorte vorgesehen sein. Der Zielspeicherbereich und der (zumindest eine) weitere Speicherort können jeweils einer Verarbeitungsstufe der Datenverarbeitungshardware, insbesondere zumindest eines Grafikprozessors der Datenverarbeitungshardware zugeordnet sein. Es kann vorgesehen sein, die Teildaten nach jeder Verarbeitungsstufe in einen neuen Speicherort zu überführen. Insbesondere kann es vorgesehen sein, einzelne Zwischenergebnisse nur so lange in einem jeweiligen Speicherort vorzuhalten, wie sie für die Berechnungen der jeweiligen Verarbeitungsstufe benötigt werden. Entsprechend einem Ringpuffer können die jeweiligen Daten anschließend durch weitere Daten aus der vorherigen Verarbeitungsstufe oder aus der Ausgabeschnittstelle des Bildgebungsgeräts überschrieben werden. Beispielsweise kann es vorgesehen sein, die Teildaten spätestens nach Berechnung eines Rebinnings an den weiteren Speicherort zu übertragen. Das Bildgebungssystem kann dazu ausgestaltet sein, den Zielspeicherbereich spätestens nach Berechnung des Rebinnings mit weiteren Teildaten zu überschreiben. Es kann auch vorgesehen sein, bereits nach einer Dekomprimierung der Teildaten diese an einen weiteren Speicherort der Datenverarbeitungshardware weiterzugeben. Alternativ kann es vorgesehen sein die Teildaten im Laufe oder nach dem Durchlaufen der Prep-Kette an einen weiteren Speicherort der Datenverarbeitungshardware weiterzugeben. Insbesondere kann, je kürzer die benötigte Prozessierungsdauer an dem Zielspeicher ist, auch die Anzahl der verfügbaren Zielspeicherbereiche für die Datenübertragung geringer dimensioniert werden. Durch eine derartige Verkettung, indem insbesondere die Speicherorte als Ringpuffer benutzt werden können, kann eine komplette Verarbeitungspipeline innerhalb der Datenverarbeitungshardware, insbesondere innerhalb eines Grafikprozessors, bei einem reduzierten Speicherbedarf erreicht werden. Die Verarbeitungsarchitektur kann somit noch effizienter gestaltet werden.

Gemäß einer Ausführungsform ist die zumindest eine Ausgabeschnittstelle mit den Zielspeicherbereichen bzw. dem zumindest einen Zielspeicher der Datenverarbeitungshardware über einen Remote Direct Memory Access (RDMA) verbunden. Eine RDMA-Verbindung zwischen der Ausgabeschnittstelle und den Zielspeicherbereichen kann insbesondere eine Möglichkeit sein, dass die Teildaten ohne Einfluss von einer weiteren Steuersoftware in den jeweiligen Zielspeicherbereich übertragen werden. Insbesondere kann die Übertragung beispielsweise ohne direkten Einfluss einer CPU vorgesehen sein. Insbesondere können somit CPU-bedingte Engpässe umgangen werden. Mit RDMA kann eine besonders unmittelbare Verkettung der Ausgabeschnittstelle, beispielsweise von anwendungsspezifischen integrierten Schaltungen (ASIC), mit den Zielspeicherbereichen, beispielsweise zumindest eine Grafikprozessors, ermöglicht werden. Die RDMA-Verbindung kann beispielsweise als RoCE (RDMA over converged Ethernet) ausgestaltet sein. Es kann vorgesehen sein, Resend-Buffer-Mechanismen einzusetzen. Dazu können im Stand der Technik gängige Resend-Buffer-Mechanismen verwendet werden. Damit kann das System robuster gegen eventuelle Störungen in der Datenübertragung sein. Beispielsweise kann RDMA in einem "reliable connected mode" betrieben werden, um insbesondere bereits protokollimmanente Sicherungsmechanismen zu verwenden. Ein weiterer Vorteil von RDAM kann sein, dass insbesondere alles, was in einem OSI-Modell unter Schicht 4 (die Transportschicht gemäß dem OSI-Modell nach der ISO) passiert, durch standardisierte Hardware erreicht werden kann. Beispielsweise muss eine Decodierung nicht mehr auf einer CPU stattfinden, sondern kann direkt in einem Grafikprozessor, umfassend zumindest einer der Zielspeicher, erfolgen. Der Adressierungsalgorithmus kann, beispielsweise im Rahmen von RDMA-Read/Write-Operationen, auf einer Senderseite, insbesondere auf dem Bildgebungsgerät, vorgesehen sein. Alternativ kann der Adressierungsalgorithmus auf einer Empfängerseite, beispielsweise im Rahmen von RDMA-Send/Receive-Operationen, z.B. auf der Verarbeitungshardware, vorgesehen sein. Somit kann insbesondere eine senderseitige Adressierung oder eine empfängerseitige Adressierung vorgesehen sein. Bei der empfängerseitigen Adressierung kann es vorgesehen sein, dass der Empfänger für einkommende Nachrichten entsprechende Speicherbereiche in sogenannten Receive-Queues hinterlegt. Insbesondere kann der Adressierungsalgorithmus dazu konfiguriert sein, diese Funktion auszuführen. Eintreffende Nachrichten können dann insbesondere ohne ein Zutun einer CPU an die hinterlegten Zielspeicherbereiche geschrieben werden. Somit kann es möglich sein, dass der Sender selbst, insbesondere auf Seiten des Bildgebungsgeräts, die konkrete Speicher-Zieladresse nicht kennt, sondern nur der Empfänger. Entsprechend kann sich bei dieser Option der Adressierungsalgorithmus primär auf der Empfängerseite befinden. Diese Variante kann eine Synchronisationsmechanismen, z.B. das Vermeiden von Überschreiben von noch nicht abgearbeiteten Speicherbereichen, erleichtern. Die Option, gemäß der der Adressierungsalgorithmus auf Senderseite vorgesehen ist, kann vorteilhafterweise eine bereits noch frühzeitigere Adressierung ermöglichen, womit auf Empfängerseite Verarbeitungsdauer und -leistung eingespart werden kann.

Gemäß einer Ausführungsform ist die Übertragung von der zumindest einen Ausgabeschnittstelle zu den Zielspeicherbereichen über eine Gather/Scatter-Adressierung vorgesehen. Mit der Gather/Scatter-Adressierung können die Teildaten eingesammelt werden (Gather) und nach der Übertragung an die gewünschten Zielspeicherbereich in der Datenverarbeitungshardware, insbesondere auf zumindest einem Grafikprozessor, verteilt werden (Scatter). Insbesondere kann die Scatter-Option eine Verteilung auf nicht zusammenhängende Zielspeicherbereiche ermöglichen. Durch Nutzung der Gather/Scatter-Adressierung kann eine effiziente und konzeptionell schlanke Verknüpfung der Erfassungsmodule mit der Datenverarbeitungshardware, insbesondere den Verarbeitungsstufen der Datenverarbeitungshardware, erreicht werden. Es können zusätzlich ansonsten eventuell nötige Umsortierungen eingespart werden. Insbesondere können die gemessenen Teildaten beispielsweise in einem einzigen Schritt zu den Zielspeicherbereichen in der der Datenverarbeitungshardware gelangen, so dass eine Weiterverarbeitung mit einer möglichst geringen Anzahl an Transfers zwischen Speichern bzw. Speicherbereichen erfolgen kann. Eine Scatter-Operation kann insbesondere mit einer empfängerseitigen Adressierung umgesetzt werden.

Gemäß einer Ausführungsform ist das Bildgebungssystem dazu ausgestaltet, die Teildaten über eine Mehrzahl von Datenkanälen, insbesondere über einen Datenkanal pro integriertem Schaltkreis (insbesondere ASIC), der jeweils einem Erfassungsmodul zugeordnet ist, von der zumindest einen Ausgabeschnittstelle zu den Zielspeicherbereichen zu senden. Diese Ausführungsform kann insbesondere mit einer RDMA-Verbindung verknüpft bzw. umgesetzt werden. Ein Aufteilen in eine Mehrzahl von Datenkanälen erlaubt ein flexibleres Datenrouting und ein besseres Loadbalancing der einzelnen Datenströme. Mit kleinen Datenkanälen kann zudem Hardware eingespart werden. Beispielsweise kann eine langsamere CPU verwendet werden, da diese lediglich die Daten der einzelnen, bereits durch den Adressierungsalgorithmus adressierten, Datenströme weiterleiten muss. Durch die Mehrzahl von Datenkanälen wird eine hohe Segmentierung der Datenübertragung, indem viele kleine Datenpakete statt einzelne große Datenpakete geschickt werden, ermöglicht. Eine hohe Segmentierung hat für die Datenübertragung den wesentlichen Vorteil, dass ein Resend oder eine Dateninterpolation auf einem wesentlich geringeren Datenvolumen erfolgen. Dadurch können bei gleicher Funktionalität die Komplexität, der Raumbedarf, der Speicherbedarf, der Bandbreitenbedarf, den Energiebedarf, die Latenz und somit auch die Kosten reduziert werden.

Gemäß einer Ausführungsform umfasst das Bildgebungssystem einen Datenspeicher zum Speichern von Messdaten, wobei das Bildgebungssystem und/oder die zumindest eine Ausgabeschnittstelle dazu konfiguriert ist, die Messdaten jeweils zusätzlich zu dem zumindest einen Datenspeicher zu senden, wobei der Datenspeicher dazu konfiguriert ist, die Messdaten zu speichern. Beispielsweise kann das Bildgebungssystem dazu ausgestaltet sein, die Daten per Multicast an den zusätzlichen Datenspeicher zu leiten. Der zusätzliche Datenspeicher kann beispielsweise ein, insbesondere direkt adressierbarer, NVMe-Speicher (NVM-Express-Speicher) sein. Insbesondere können die Messdaten somit, beispielsweise für eine spätere Weiterverarbeitung oder Überprüfung, auch als Rohdaten gespeichert werden, insbesondere parallelisiert zu der Verarbeitung der Messdaten mit der Datenverarbeitungshardware. Der Datenspeicher kann Teil des Bildgebungssystems sein. Der Datenspeicher kann optional ein Remote-Speicher sein. Beispielsweise kann der Datenspeicher auch über ein Netzwerk und/oder das Internet mit dem Bildgebungssystem verbunden sein.

Alternativ oder zusätzlich kann das Bildgebungssystem dazu ausgestaltet sein, teilweise von der Datenverarbeitungshardware verarbeitete Daten zu extrahieren und an den Datenspeicher zu senden. Teilweise von der Datenverarbeitungshardware verarbeitete Daten können beispielsweise dekomprimierte Daten, gepreppte bzw. vorverarbeitete Daten, Daten nach einem Rebinning und/oder Daten nach einer Faltung sein. Alternativ oder zusätzlich kann das Bildgebungssystem dazu ausgestaltet sein, von der Datenverarbeitungshardware vollständig verarbeitete Daten, beispielsweise rekonstruierte Bilddaten, an den Datenspeicher zu senden. Rekonstruierte Bilddaten können beispielsweise Schnittbilder bzw. ein Satz von Schnittbildern und/oder dreidimensionale Bilddaten sein.

Ein weiterer Aspekt der Erfindung ist ein Bildgebungsgerät, insbesondere wie hierin beschrieben. Das Bildgebungsgerät kann insbesondere ein Computertomographiegerät, ein Positronen-Emissions-Tomographie-Gerät, ein Fluoroskopie-Gerät und/oder ein Ultraschall-Gerät sein. Das Bildgebungsgerät umfasst eine Erfassungsvorrichtung zum Erfassen und Weiterleiten von Messdaten, wobei die Erfassungsvorrichtung zumindest ein Erfassungsmodul, insbesondere eine Mehrzahl von Erfassungsmodulen, zum Erfassen von Teildaten, die jeweils ein Teil der gesamten Messdaten sind, umfasst, wobei das Bildgebungsgerät dazu konfiguriert ist, mit Hilfe des zumindest einen Erfassungsmoduls Gruppen von Teildaten zu erzeugen, wobei jede der Gruppen Teildaten von zumindest einem des zumindest einen Erfassungsmodul umfasst. Optional umfasst das Bildgebungsgerät zumindest einen Adressierungsalgorithmus, der dazu konfiguriert ist, basierend auf Informationen über die Struktur einer Datenverarbeitungshardware mit einer Mehrzahl von Zielspeicherbereichen, optional verteilt auf mehrere Zielspeicher, die Gruppen jeweils mit einer Zieladresse betreffend jeweils einen der Zielspeicherbereiche in der Datenverarbeitungshardware zu verknüpfen. Alternativ kann der Adressierungsalgorithmus außerhalb des Bildgebungsgeräts, beispielsweise auf der Datenverarbeitungshardware, vorgesehen sein. Das Bildgebungsgerät umfasst zumindest eine Ausgabeschnittstelle zum Weiterleiten der mit jeweils zumindest einer Zieladresse verknüpften Gruppen von Teildaten in Richtung zumindest des der Zieladresse entsprechenden Zielspeicherbereichs. Alle Vorteile und Merkmale des Bildgebungssystems können analog auf das Bildgebungsgerät übertragen werden und umgekehrt.

Ein weiterer Aspekt der Erfindung ist ein Verfahren zum Übertragen von Messdaten von einer Erfassungsvorrichtung eines Bildgebungsgeräts mit zumindest einem Erfassungsmodul, insbesondere einer Mehrzahl von Erfassungsmodulen, zu einer Datenverarbeitungshardware mit einer Mehrzahl von Zielspeicherbereichen, optional verteilt auf mehrere Zielspeicher, umfassend die folgenden Schritte:

- Erzeugen von Gruppen von Teildaten der Messdaten des zumindest einen Erfassungsmoduls;
- Verknüpfen der Gruppen mit jeweils einer Zieladresse betreffend jeweils einen der Zielspeicherbereiche, vorzugsweise durch einen Adressierungsalgorithmus, insbesondere basierend auf Informationen über die Struktur der Datenverarbeitungshardware mit der Mehrzahl von Zielspeicherbereichen;
- Weiterleiten der Gruppen von Teildaten zu den Zielspeicherbereichen, wobei die Teildaten in der Datenverarbeitungshardware anhand der Zieladressen direkt den jeweiligen Zielspeicherbereichen zugeordnet werden.

Alle Vorteile und Merkmale des Bildgebungsgeräts und des Bildgebungssystems können analog auf das Verfahren zum Übertragen von Messdaten übertragen werden und umgekehrt.

Ein weiterer Aspekt der Erfindung ist ein Verfahren zum Verarbeiten von aufgenommenen Messdaten. Das Verfahren zum Weiterverarbeiten von Messdaten kann die Schritte des Verfahrens zum Übertragen von Messdaten umfassen und zusätzlich den Schritt aufweisen:

- Verarbeiten, insbesondere Rekonstruieren, der Messdaten in der Datenverarbeitungshardware.

Alle Vorteile und Merkmale des Bildgebungsgeräts, des Bildgebungssystems und des Verfahrens zum Übertragen von Messdaten können analog auf das Verfahren zum Verarbeiten von aufgenommenen Messdaten übertragen werden und umgekehrt. Das Verarbeiten kann insbesondere auf zumindest einem Grafikprozessor der Datenverarbeitungshardware vorgesehen sein. Das Verarbeiten kann ein Dekomprimieren der Teildaten umfassen. Das Verarbeiten kann ein Vorverarbeiten der Teildaten in einer Vorverarbeitungsstufe umfassen. Das Vorverarbeiten kann ein Anwenden von Korrekturen auf die Teildaten umfassen. Nach dem Vorverarbeiten ist insbesondere zumindest ein weiterer Verarbeitungsschritt, insbesondere umfassend eine Bildrekonstruktion, vorgesehen. Das Verarbeiten kann ein Rebinning der Teildaten umfassen, insbesondere zum Überführen der in der Vorverarbeitungsstufe vorbereiteten Daten in zumindest einen Satz von Projektionen. Das Verarbeiten kann ein Falten der Projektionen mit einem Filterkern umfassen. Das Verarbeiten kann ein schrittweises Verwenden der gefalteten Daten durch einen Backprojektor für die Berechnung von Bilddaten, insbesondere Schnittbildern umfassen. Ein abschließender Schritt des Verarbeitens kann ein Ablegen der verarbeiteten Daten, beispielsweise als DICOM-Daten, umfassen. Vorzugsweise werden einzelne Zwischenergebnisse des Verarbeitens nur so lange vorgehalten, wie sie für die Berechnungen des jeweiligen Verarbeitungsschritts benötigt werden. Insbesondere werden die Daten jeder Stufe vorzugsweise nach dem Abschließen jedes Verarbeitungsschritts durch neue Daten aus der vorherigen Stufe bzw. durch die Teildaten aus den Erfassungsmodulen der Erfassungsvorrichtung überschrieben.

Alle hierin beschriebenen Ausführungsformen können miteinander kombiniert werden, soweit nicht explizit etwas anderes angegeben ist.

Im Folgenden werden Ausführungsformen mit Bezug auf die beigefügten Figuren beschrieben.

Fig. 1    zeigt ein Schema eines Bildgebungssystems gemäß einer Ausführungsform der Erfindung,

Fig. 2    zeigt ein Flussdiagramm eines Verfahrens zum Übertragen von Messdaten von einer Erfassungsvorrichtung eines Bildgebungsgeräts mit einer Mehrzahl von Erfassungsmodulen zu einer Datenverarbeitungshardware mit einer Mehrzahl von Zielspeicherbereichen gemäß einer Ausführungsform der Erfindung, und

Fig. 3    zeigt ein Flussdiagramm eines Verfahrens zum Verarbeiten von aufgenommenen Messdaten gemäß einer Ausführungsform der Erfindung.

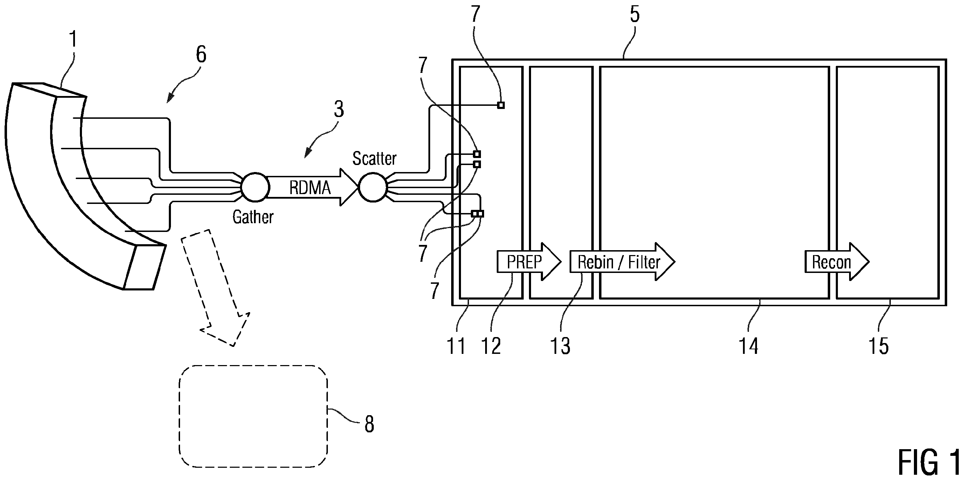

Figur 1 zeigt ein Schema eines Bildgebungssystems gemäß einer Ausführungsform der Erfindung. Das Bildgebungssystem ist in diesem Ausführungsbeispiel ein Computertomographiesystem mit einer rotierbaren Gantry (nicht gezeigt). Die Gantry umfasst eine Erfassungsvorrichtung 1, die aus teilkreisförmig angeordneten Erfassungsmodulen zur Detektion von Röntgenstrahlung besteht. Das Prinzip der Erfindung kann jedoch analog auch für andere Arten von Bildgebungssystemen verwendet werden. Die Erfassungsmodule der Erfassungsvorrichtung 1 umfassen jeweils anwendungsspezifischen integrierten Schaltungen (ASICs), in denen eingehende Signale elektronisch aufgenommen und weitergeleitet werden.

Bereits in dem jeweiligen ASIC, alternativ in einer unmittelbar nachgelagerten Stufe, der Erfassungsmodule der Erfassungsvorrichtung 1 werden die Messdaten, beispielsweise spektralen Messdaten, eines oder mehrerer Frames in geeigneter Weise als Teildaten zusammengefasst und komprimiert. Die nachgelagerte Stufe kann beispielsweise ein dedizierter ASIC, ein SoC (System-on-Chip), ein SiP (System-in-Package), ein FPGA, ein DSP, eine CPU oder etwas Entsprechendes sein. Mit einem Adressierungsalgorithmus, der jeweils in den ASICs vorgesehen ist, werden die jeweiligen Teildaten mit einer Zieladresse 7 betreffend einen Zielspeicherbereich verknüpft. Dabei wird, basierend auf einer in dem ASIC hinterlegten oder von extern abgerufenen Position [X, Y] des ASICs in der Erfassungsvorrichtung 1 sowie auf der Lage des Erfassungsvorrichtung 1, 8, mit dem Adressierungsalgorithmus eine geeignete Zieladresse 7, A, im Speicher eines Grafikprozessors 5 (GPU) der Bildverarbeitungshardware ermittelt. Gibt es mehrere Grafikprozessoren 5, bestimmt der Adressierungsalgorithmus auch die GPUID der relevanten Ziel-GPU. Durch die Positionsabhängigkeit der Adresse kann erreicht werden, dass die Teildaten an einen für die Weiterverarbeitung optimiertem Zielbereich landen und dass sich die Daten aufeinanderfolgender Übertragungen nicht überschreiben. Durch den Adressierungsalgorithmus f

$$f(X, Y, \theta) = [A, GPUID]$$

kann erreicht werden, dass Daten im Speicher des Grafikprozessors 5 erst dann überschrieben werden, nachdem die Weiterverarbeitung dieses Bereiches in dem Grafikprozessor 5 abgeschlossen ist. Das geeignete Ineinandergreifen der Adressierung und der Echtzeit-Weiterverarbeitung der Blöcke in dem Grafikprozessor 5 können insbesondere bezogen auf den konkreten Einsatzzweck der Bildgebungssystems und, beispielsweise auf die vorgesehenen Messmodi, aufeinander abgestimmt werden. Ermöglicht beispielsweise der Speicher des Grafikprozessors 5 die Speicherung der Daten zweier kompletter Rotationen der Erfassungsvorrichtung 1 in der Gantry, so wird bei einer Rotationszeit von 0.2 s beispielsweise der gleiche Speicherbereich erst nach 400 ms erneut überschrieben. In diesem Zeitfenster sollte die Weiterverarbeitung dieses Blockes in dem Grafikprozessor 5 garantiert abgeschlossen sein.

Über eine Ausgabeschnittstelle werden die Teildaten als Datenpakete über eine Mehrzahl von Datenkanälen 6 versendet. Beispielsweise kann eine Übertragung über Ethernet vorgesehen sein. In diesem Ausführungsbeispiel sind die Datenpakete RDMA-adressiert und werden mittels einer RDMA-Kette (z.B. via RoCE, RDMA over Converged Ethernet) übertragen und an den Zielorten im Speicher des Grafikprozessors 5 abgeliefert. Dabei wird eine Gather/Scatter-Adressierung 3 eingesetzt, um die Teildaten der verschieden ASICs einzusammeln (Gather) und nach der Übertragung an die gewünschten Speicherbereiche auf dem Grafikprozessor 5 (oder auf den verschiedenen Grafikprozessoren 5) zu verteilen (Scatter). Alternativ oder zusätzlich zu dem Grafikprozessor 5 können die Daten beispielsweise mittels Multicast auch an einen Datenspeicher 8 (z.B. einen direkt adressierbaren NVMe Speicher) geleitet werden und dort insbesondere als Rohdaten gespeichert werden. Verzichtet man auf die Speicherung der Rohdaten auf dem Datenspeicher 8, so kann es vorgesehen sein, dass das Ergebnis der Verarbeitung in dem Grafikprozessor 5 bzw. den Grafikprozessoren 5 derart ausgestaltet ist, dass alle für die weitere Befundung benötigten Daten erhalten sind. Beispielsweise könnte das Ergebnis der Verarbeitung ein Satz hochaufgelöster (scharfer) Schnittbilder sein. Alternativ könnte auch zu einem früheren Schritt der Verarbeitung die Extraktion teilweise verarbeiteter Daten (z.B. dekomprimierter Daten, gepreppter Daten, Daten nach Rebinning oder Daten nach Faltung) vorgesehen sein. Um das System robuster gegen eventuelle Störungen in der Datenübertragung zu machen, können optional gängige Resend-Buffer-Mechanismen vorgesehen sein. Beispielsweise kann hierzu RDMA in einem "reliable connected mode " betrieben werden, wodurch protokollimmanente Sicherungsmechanismen genutzt werden können.

In dem Grafikprozessor 5 werden die Teildaten, die an den jeweiligen Zieladressen 7 ankommen, weiterverarbeitet. Idealerweise muss erst das Endergebnis (z.B. in Form von rekonstruierten Schnittbildern) aus dem Grafikprozessor 5 ausgelesen werden. Beispielsweise kann das Endergebnis als DICOM-Daten abgelegt werden. Vorteilhafterweise müssen die Daten in der Datenverarbeitungshardware nicht mehr adressiert werden, sondern können aufgrund der bereits erfolgten Adressierung mit dem Adressierungsalgorithmus direkt weiterverarbeitet werden. Dadurch kann eine deutlich schnellere und effizientere Bearbeitung in der Datenverarbeitungshardware ermöglicht werden. In diesem Ausführungsbeispiel involviert diese Weiterverarbeitung zunächst in einer Vorverarbeitungsstufe 11 eine Dekomprimierung der Daten und die Anwendung einer Prep-Kette 12. In der Prep-Kette, werden, insbesondere überwiegend lokale, Korrekturen auf die Messdaten angewendet. Die gepreppten Daten werden dann mittels einer Rebinning-Stufe 13 von, insbesondere fächerförmigen, Rohdaten in einen Satz von Projektionen 14 überführt. Die Projektionen 14 werden dann mit einem Filterkern gefaltet und die gefalteten Daten werden von einem Backprojektor schrittweise für die Berechnung der rekonstruierten Bilddaten 15, insbesondere Schnittbilder, verwendet. Beispielsweise können verschieden Sätze von Schichtbildern kombiniert und transformiert (z.B. zu VNC-, Mono-keV-, Iodine- Bildern, etc.) und gewünschte Ansichten bzw. Schnitte errechnet werden. Durch die erfindungsgemäße Struktur entsteht eine GPU-Interne Pipeline, bei der die einzelnen Zwischenergebnisse nur so lange vorgehalten werden müssen, wie sie für die Berechnungen der nachfolgenden Verarbeitungsstufe benötigt werden. Anschließend werden sie durch neue Daten aus der vorherigen Stufe oder aus dem Detektor überschrieben (Ringpuffer). Spätestens nach Berechnung der Rebinnings kann also der ursprünglich für den Datentransfer verwendete Speicherblock erneut überschieben werden. Alternativ kann auch bereits nach der Dekomprimierung oder im Laufe der Prep-Kette das Ergebnis in einem anderen Speicherbereich abgelegt werden, so dass der ursprüngliche Speicherblock früher überschrieben werden kann. Je kürzer die benötigte Prozessierungsdauer, desto geringer kann auch die Anzahl der verfügbaren Zielbereiche für die Datenübertragung dimensioniert werden. Optional können auch verschiedene Übertragungsmodi und Parametrisierungen unterstützt werden. Beispielsweise könnten sich die Modi in Rotationszeit, Framerate, Z-Abdeckung, Schwellenzahl und Ortsauflösung unterscheiden. Ferner kann es auch verschiedene System-Konfigurationen mit unterschiedlicher Anzahl, Art und Ausbaustufe der Grafikprozessoren 5 geben, auf welche der Adressierungsalgorithmus hin angepasst wird. Vorteilhafterweise kann es dazu vorgesehen sein, dass der Adressierungsalgorithmus extern parametrisierbar ausgestaltet, z.B. über Konfigurationsregister.

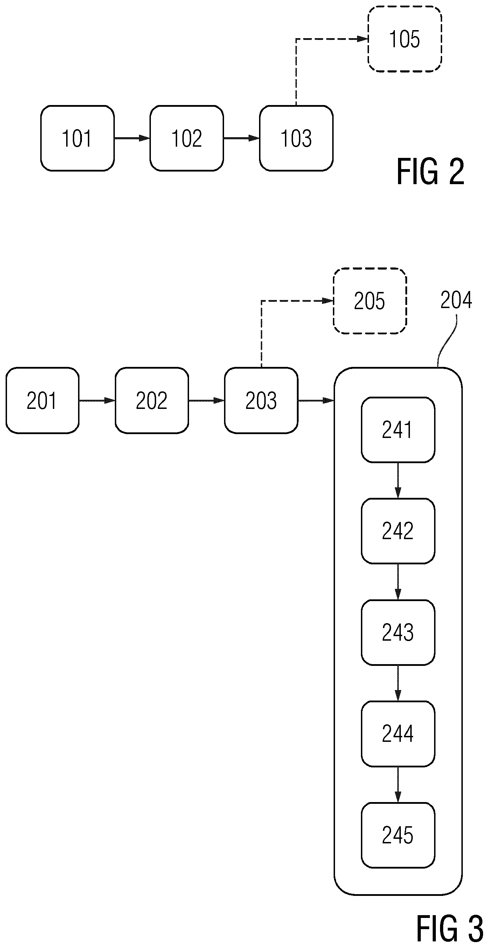

Figur 2 zeigt ein Flussdiagramm eines Verfahrens zum Übertragen von Messdaten von einer Erfassungsvorrichtung 1 eines Bildgebungsgeräts mit einer Mehrzahl von Erfassungsmodulen zu einer Datenverarbeitungshardware mit einer Mehrzahl von Zielspeicherbereichen gemäß einer Ausführungsform der Erfindung. In einem ersten Schritt 101 werden Gruppen von Teildaten der Messdaten der Erfassungsmodule erzeugt. Dabei umfasst insbesondere jede der Gruppen Teildaten von zumindest einem Erfassungsmodul. Die Gruppen können insbesondere mit einem Gruppierungsalgorithmus erzeugt werden, der dazu konfiguriert ist, die Teildaten verschiedener Erfassungsmodule und/oder verschiedener Erfassungszeitpunkte zu den Gruppen von Teildaten zusammenzufassen. Vorzugsweise können die Gruppen von Teildaten zudem von einem Komprimierungsalgorithmus komprimiert werden. In einem weiteren Schritt 102 werden die Gruppen mit jeweils einer Zieladresse 7 betreffend jeweils einen der Zielspeicherbereiche verknüpft, insbesondere basierend auf Informationen über die Struktur einer Datenverarbeitungshardware mit einer Mehrzahl von Zielspeicherbereichen. Das Verknüpfen wird insbesondere durch einen Adressierungsalgorithmus wie hierin beschrieben durchgeführt. Vorzugsweise wird das Verknüpfen in integrierten Schaltkreisen, insbesondere anwendungsspezifischen integrierten Schaltungen, der Erfassungsmodule oder in einer den integrierten Schaltkreisen unmittelbar nachgelagerten Verarbeitungsstufe in dem Bildgebungsgerät durchgeführt. Bei einer beweglichen Erfassungsvorrichtung 1, beispielsweise einer rotierbaren Erfassungsvorrichtung 1 in einer Gantry eines Computertomographiegeräts, kann es vorgesehen sein, die jeweilige Zieladresse 7 auch basierend auf der aktuellen Position der Erfassungsmodule, von denen die jeweiligen Teildaten stammen, festzulegen. Optional kann der Adressierungsalgorithmus ferner dazu konfiguriert sein, die jeweilige Zieladresse 7 auch basierend auf einer logischen und/oder physischen Position der Zielspeicherbereiche bzw. mehrerer Zielspeicher in der Datenverarbeitungshardware und/oder basierend auf Informationen über eine Verarbeitungsdauer der Datenverarbeitungshardware an dem jeweiligen Zielspeicherbereich festzulegen, sodass das erneute Senden von Teildaten in einen Zielspeicherbereich mit dem Freiwerden des Zielspeicherbereichs abgestimmt ist. In einem weiteren Schritt 103 werden die Gruppen von Teildaten zu den Zielspeicherbereichen geleitet, beispielsweise über einen Remote Direct Memory Access und mit einer Gather/Scatter-Adressierung 3, wobei die Teildaten in der Datenverarbeitungshardware anhand der Zieladressen 7 direkt den jeweiligen Zielspeicherbereichen zugeordnet werden. In einem optionalen Schritt 105 kann es zudem vorgesehen sein, die Messdaten jeweils zusätzlich zu zumindest einem Datenspeicher 8 zu senden.

Figur 3 zeigt ein Flussdiagramm eines Verfahrens zum Verarbeiten von aufgenommenen Messdaten gemäß einer Ausführungsform der Erfindung. Die Schritte 201-203 und der optionale Schritt 205 können den Schritten 101-103 und 105 des mit Bezug auf Figur 2 beschriebenen Verfahrens entsprechen. In einem weiteren Schritt 204 werden die Messdaten in der Datenverarbeitungshardware verarbeitet, insbesondere zu Bilddaten 15 rekonstruiert. Der weitere Schritt 204 umfasst mehrere Teilschritte. In einem (optionalen) ersten Teilschritt 241 werden die Teildaten dekomprimiert, falls sie zuvor komprimiert wurden. In einem weiteren Teilschritt 242, wird eine Prep-Kette angewandt, bei der überwiegend lokale Korrekturen auf die Teildaten angewendet werden. In einem weiteren Teilschritt 243 werden die gepreppten Daten durch ein Rebinning in einen Satz von Projektionen 14 überführt. Die Projektionen 14 werden dann in einem weiteren Teilschritt 244 mit einem Filterkern gefaltet. In einem weiteren Schritt 245 werden die gefalteten Daten werden von einem Backprojektor schrittweise für die Berechnung der rekonstruierten Bilddaten 15, insbesondere Schnittbilder, verwendet.

**Patentansprüche**

1.  Bildgebungssystem umfassend ein Bildgebungsgerät,

    wobei das Bildgebungsgerät eine Erfassungsvorrichtung (1) zum Erfassen und Weiterleiten von Messdaten umfasst,
    wobei die Erfassungsvorrichtung (1) zumindest ein Erfassungsmodul zum Erfassen von Teildaten, die jeweils ein Teil der gesamten Messdaten sind, umfasst,
    wobei das Bildgebungsgerät dazu konfiguriert ist, mit Hilfe des zumindest einen Erfassungsmoduls Gruppen von Teildaten zu erzeugen,
    wobei jede der Gruppen Teildaten von zumindest einem des zumindest einen Erfassungsmoduls umfasst;

    wobei das Bildgebungssystem zumindest einen Adressierungsalgorithmus umfasst, der dazu konfiguriert ist, basierend auf Informationen über eine Struktur einer Datenverarbeitungshardware mit einer Mehrzahl von Zielspeicherbereichen die Gruppen jeweils mit einer Zieladresse (7) betreffend jeweils einen der Zielspeicherbereiche in der Datenverarbeitungshardware zu verknüpfen;
    wobei das Bildgebungsgerät zumindest eine Ausgabeschnittstelle zum Weiterleiten der mit jeweils zumindest einer Zieladresse (7) verknüpften Gruppen von Teildaten in Richtung zumindest des der Zieladresse (7) entsprechenden Zielspeicherbereichs umfasst.

2.  Bildgebungssystem gemäß Anspruch 1,

    wobei das Bildgebungsgerät eine Mehrzahl von integrierten Schaltkreisen umfasst, die jeweils zumindest einem Erfassungsmodul zugeordnet sind und dazu konfiguriert sind, Teildaten von dem zumindest einen zugeordneten Erfassungsmodul zu verarbeiten,
    wobei die Mehrzahl von integrierten Schaltkreisen jeweils einen des zumindest einen Adressierungsalgorithmus umfassen, oder wobei der zumindest eine Adressierungsalgorithmus Teil zumindest einer den integrierten Schaltkreisen unmittelbar nachgelagerten Verarbeitungsstufe in dem Bildgebungsgerät ist.

3.  Bildgebungssystem gemäß einem der vorhergehenden Ansprüche,
    wobei das Bildgebungsgerät zumindest einen Gruppierungsalgorithmus umfasst, der dazu konfiguriert ist, die Teildaten verschiedener Erfassungsmodule und/oder verschiedener Erfassungszeitpunkte zu den Gruppen von Teildaten zusammenzufassen.

4.  Bildgebungssystem gemäß einem der vorhergehenden Ansprüche,
    wobei das Bildgebungsgerät zumindest einen Komprimierungsalgorithmus umfasst, der dazu konfiguriert ist, Gruppen von Teildaten zu komprimieren bevor diese an die Ausgabeschnittstelle übergeben werden.

5.  Bildgebungssystem gemäß einem der vorhergehenden Ansprüche,

    wobei die Erfassungsvorrichtung (1) dazu ausgestaltet ist, während der Erfassung von Messdaten bewegt zu werden, insbesondere rotiert zu werden,
    wobei der zumindest eine Adressierungsalgorithmus ferner dazu konfiguriert ist, die jeweilige Zieladresse (7) auch basierend auf der aktuellen Position der Erfassungsmodule, von denen die jeweiligen Teildaten stammen, festzulegen.

**6.** Bildgebungssystem gemäß Anspruch 5, wobei der Adressierungsalgorithmus ferner dazu konfiguriert ist, die jeweilige Zieladresse (7) auch basierend auf einer logischen und/oder physischen Position der Zielspeicherbereiche in der Datenverarbeitungshardware festzulegen.

**7.** Bildgebungssystem gemäß einem der vorhergehenden Ansprüche, wobei der zumindest eine Adressierungsalgorithmus dazu konfiguriert ist, die Zieladressen (7) jeweils auch basierend auf Informationen über eine Verarbeitungsdauer der Datenverarbeitungshardware an dem jeweiligen Zielspeicherbereich festzulegen, sodass das erneute Senden von Teildaten in einen Zielspeicherbereich mit dem Freiwerden des Zielspeicherbereichs abgestimmt ist.

**8.** Bildgebungssystem gemäß einem der vorhergehenden Ansprüche,

wobei das Bildgebungsgerät und der Adressierungsalgorithmus so konfiguriert sind, dass der Adressierungsalgorithmus durch eine Eingabe parametrisierbar ist, wobei die Parametrisierbarkeit insbesondere in Abhängigkeit von vorgesehenen Messparametern eines Messprotokolls vorgesehen ist.

**9.** Bildgebungssystem gemäß einem der vorhergehenden Ansprüche umfassend eine Datenverarbeitungshardware mit einer Mehrzahl von Zielspeicherbereichen, optional verteilt auf mehrere Zielspeicher, wobei die Datenverarbeitungshardware insbesondere zumindest einen Grafikprozessor (5) umfasst, wobei die Datenverarbeitungshardware mit der zumindest einen Ausgabeschnittstelle datentechnisch verbunden und/oder verbindbar ist und dazu konfiguriert ist, die Gruppen von Teildaten zu verarbeiten, insbesondere im Rahmen einer Bildrekonstruktion.

**10.** Bildgebungssystem gemäß Anspruch 9,

wobei die Datenverarbeitungshardware dazu konfiguriert ist, die Gruppen von Teildaten anhand der verknüpften Zieladressen (7) direkt an den zugehörigen Zielspeicherbereich zu leiten und die Teildaten an dem Ort des Zielspeicherbereichs zu bearbeiten, wobei die Datenverarbeitungshardware optional dazu konfiguriert ist, die Teildaten nach der Bearbeitung in dem Zielspeicherbereich an einen weiteren Speicherort der Datenverarbeitungshardware weiterzugeben und dort weiterzubearbeiten.

**11.** Bildgebungssystem gemäß einem der Ansprüche 9 oder 10, wobei die zumindest eine Ausgabeschnittstelle mit den Zielspeicherbereichen der Datenverarbeitungshardware über einen Remote Direct Memory Access verbunden ist.

**12.** Bildgebungssystem gemäß Anspruch 11, wobei die Übertragung von der zumindest einen Ausgabeschnittstelle zu den Zielspeicherbereichen über eine Gather/Scatter-Adressierung (3) vorgesehen ist.

**13.** Bildgebungssystem gemäß einem der Ansprüche 9 bis 12, wobei das Bildgebungssystem dazu ausgestaltet ist, die Teildaten über eine Mehrzahl von Datenkanälen (6), insbesondere über einen Datenkanal (6) pro integriertem Schaltkreis, der jeweils einem Erfassungsmodul zugeordnet ist, von der zumindest einen Ausgabeschnittstelle zu den Zielspeicherbereichen zu senden.

**14.** Bildgebungssystem gemäß einem der Ansprüche 9 bis 13, wobei das Bildgebungssystem einen Datenspeicher zum Speichern von Messdaten umfasst, wobei das Bildgebungssystem und/oder die zumindest eine Ausgabeschnittstelle dazu konfiguriert ist, die Messdaten jeweils zusätzlich zu dem zumindest einen Datenspeicher zu senden, wobei der Datenspeicher dazu konfiguriert ist, die Messdaten zu speichern.

**15.** Verfahren zum Übertragen von Messdaten von einer Erfassungsvorrichtung (1) eines Bildgebungsgeräts mit zumindest einem Erfassungsmodul, insbesondere einer Mehrzahl von Erfassungsmodulen zu einer Datenverarbeitungshardware mit einer Mehrzahl von Zielspeicherbereichen, optional verteilt auf mehrere Zielspeicher, umfassend die folgenden Schritte:

- Erzeugen von Gruppen von Teildaten der Messdaten des zumindest einen Erfassungsmoduls;
- Verknüpfen der Gruppen mit jeweils einer Zieladresse (7) betreffend jeweils einen der Zielspeicherbereiche durch einen Adressierungsalgorithmus, insbesondere basierend auf Informationen über die Struktur der Datenverarbeitungshardware mit der Mehrzahl von Zielspeicherbereichen;
- Weiterleiten der Gruppen von Teildaten zu den Zielspeicherbereichen, wobei die Teildaten in der Datenverarbeitungshardware anhand der Zieladressen direkt den jeweiligen Zielspeicher-

25

**EP 4 636 604 A1**

26

bereichen zugeordnet werden.

FIG 1

EP 4 636 604 A1

FIG 2

FIG 3

Europäisches
Patentamt

European
Patent Office

Office européen
des brevets

# EUROPÄISCHER RECHERCHENBERICHT

Nummer der Anmeldung

EP 24 17 0816

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2023/110667 A1 (BEGELMAN JAMES LAWRENCE [US] ET AL) 13. April 2023 (2023-04-13) | 1-4,6, 8-15 | INV. G06F13/12 |
| A | * Abbildungen 1,2,3,5c,6,7,8 * * Absätze [0002], [0015], [0025], [0026], [0027], [0029], [0030], [0039] * * Absatz [0037] * ----- | 5,7 | G06F13/16 G06F13/28 G06F13/40 A61B6/00 A61B8/08 G06T11/00 |
| A | US 2016/267653 A1 (SASAYA TOMOTAKA [JP]) 15. September 2016 (2016-09-15) * Abbildungen 1,2,5,7a, 7b, 8 * * Absatz [0042] - Absatz [0051] * ----- | 1-15 | A61B6/03 G01T1/29 |
| A | US 2009/169119 A1 (WEGENER ALBERT W [US]) 2. Juli 2009 (2009-07-02) * Abbildungen 2a,2b,10 * * Absätze [0042], [0043] * * Absatz [0066] * ----- | 1-15 | |

### RECHERCHIERTE SACHGEBIETE (IPC)

G06F
A61B
G06T
G01V
G01T

Der vorliegende Recherchenbericht wurde für alle Patentansprüche erstellt

| Recherchenort | Abschlußdatum der Recherche | Prüfer |
|---|---|---|
| München | 13. September 2024 | Nold, Michael |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
.......................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**  EP 24 17 0816

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-09-2024

| Im Recherchenbericht angeführtes Patentdokument | Datum der Veröffentlichung | Mitglied(er) der Patentfamilie | Datum der Veröffentlichung |
|---|---|---|---|
| US 2023110667 A1 | 13-04-2023 | JP 2023058006 A<br>US 2023110667 A1 | 24-04-2023<br>13-04-2023 |
| US 2016267653 A1 | 15-09-2016 | KEINE | |
| US 2009169119 A1 | 02-07-2009 | CA 2706459 A1<br>CN 101883525 A<br>EP 2217149 A2<br>JP 5460612 B2<br>JP 2011505228 A<br>KR 20100099252 A<br>US 2009169119 A1<br>WO 2009073730 A2 | 11-06-2009<br>10-11-2010<br>18-08-2010<br>02-04-2014<br>24-02-2011<br>10-09-2010<br>02-07-2009<br>11-06-2009 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010063435 A1